# EUROPEAN PATENT APPLICATION

(11) **EP 1 619 503 A1**
(43) Date of publication of application: **25.01.2006**
(21) Application number: 04016696.9
(22) Date of filing: 15.07.2004
(51) Int. Cl.: G01N 33/68, G01N 33/48

(54) **Use of standards for monitoring alterations of peptide and protein samples**

(71) Applicant: BioVisioN AG, 30625 Hannover (DE)
(72) Inventor: Tammen, Harald, 30449 Hannover (DE); Schulz-Knappe, Peter, 30966 Hemmingen (DE); Lamping, Norbert, 30175 Hannover (DE); Zucht, Hans-Dieter, 30539 Hannover (DE); Hess, Rüdiger, 30629 Hannover (DE); Kellmann, Markus, 27243 Harpstedt (DE); Menzel, Christoph, 32427 Minden (DE); Schulte, Imke, 30161 Hannover (DE)
(74) Representative: Schnappauf, Georg

(57) **Abstract**

The present invention is directed to methods of using standards for determining and monitoring alterations and variations of peptide and protein samples. More specifically, the present invention relates to methods of determining and monitoring alterations of the sample quality, in particular after collection, during processing and during storage of the sample.

## Description

### Background of the invention

An important aspect of samples comprising biological materials is their stability and quality. Since proteins and peptides are prone to proteolytic degradation and modification the stability and quality of peptide and protein samples may change between different samples and during storage. This holds in particular true for peptide and protein samples which are sensitive to proteolytic activity, modification and/or peptide loss and for samples such as biological samples which are known or suspected to contain factors having such detrimental activities on peptides and proteins contained in the sample.

Thus, there is a need for means which are useful for determining, controlling and monitoring the quality of peptide and protein samples.

### Field of the invention

The present invention is directed to methods of using standards for determining and monitoring alterations and variations of peptide and protein samples. More specifically, the present invention relates to methods of determining and monitoring alterations of the sample quality, in particular after collection, during processing and during storage of the sample. According to the invention the sample quality is determined or monitored by determining or monitoring alterations of standards contained in the sample thus providing a measure for the quality of the peptides and/or proteins in the sample. This allows for example the determination whether the sample is suitable for biomarker detection, or biomarker or therapeutics discovery by analyzing the peptide composition of the sample.

### Summary of the invention

In one aspect the invention relates to a method for determining, monitoring and/or controlling the quality of a sample comprising at least one peptide and/or protein comprising the steps of providing at least one protease-sensitive standard and/or at least one modification-sensitive standard and/or at least one standard for monitoring peptide loss, adding said sample comprising at least one peptide and/or protein, and determining the level of proteolytic cleavage and/or modification and/or peptide loss of said standard.

In a further aspect the invention relates to a method for determining, monitoring and/or controlling the quality of a sample comprising at least one peptide and/or protein comprising the steps of providing said sample comprising at least one peptide and/or protein, adding at least one protease-sensitive standard and/or at least one modification-sensitive standard and/or at least one standard for monitoring peptide loss, and determining the level of proteolytic cleavage and/or modification and/or peptide loss of said standard.

The term "level of proteolytic cleavage and/or modification and/or peptide loss" according to the present invention relates to the degree of proteolytic cleavage and/or modification and/or peptide loss determined in the standard. The term includes situations where no proteolytic cleavage and/or modification and/or peptide loss of the standard is detectable preferably indicating that no such activity is present in the sample or situations in which proteolytic cleavage and/or modification and/or peptide loss of the standard is detectable preferably indicating that such activity is present in the sample and allowing to extrapolate the level of proteolytic cleavage and/or modification and/or peptide loss determined for the standard to the components of a sample. Regarding different samples, the level of proteolytic cleavage and/or modification and/or peptide loss may be different, i.e. a different degree of proteolytic cleavage and/or modification and/or peptide loss of the standard may be present in different samples or there may be no proteolytic cleavage and/or modification and/or peptide loss present in one or more samples and a certain degree of proteolytic cleavage and/or modification and/or peptide loss may be present in other samples which degree could differ between different samples or between different groups of samples.

In preferred embodiments of the invention the at least one standard comprises at least one peptide and/or protein which is sensitive to proteolytic activity and/or modification and/or peptide loss.

In an embodiment of the methods of the invention the proteolytic cleavage and/or modification and/or peptide loss is determined at least at a second point of time. In a more preferred embodiment the progression of proteolytic cleavage and/or modification and/or peptide loss is determined by comparing the level of proteolytic cleavage and/or modification and/or peptide loss between said first and second or further determination. This embodiment allows the the determination of the progression of proteolytic cleavage and/or the level of modification and/or the peptide loss of said standard and thus, of the peptide and/or protein in the sample with time.

In still further embodiments the methods of the invention comprise the steps of determining the level of proteolytic cleavage and/or modification and/or peptide loss of the standards in at least two different samples and comparing said levels.

Preferably the modification of the at least one standard is due to the presence of at least one enzyme selected from the group consisting of glycosidases, phosphatases, kinases, lipases and nucleases, or due to the presence of at least one protease selected from the group consisting of serine proteases, cysteine proteases, threonine proteases, aspartic proteases, glutamic proteases, thiol proteases, metalloproteases, exoproteases, endoproteases, proteases involved in the clotting cascade and proteases involved in the classical and/or alternative complement cascade.

In preferred embodiments the at least one standard is at least one protease sensitive standard which preferably comprises at least one peptide and/or protein comprising at least one cleavage site for at least one protease, preferably selected from the group consisting of serine proteases, cysteine proteases, threonine proteases, aspartic proteases, glutamic proteases, thiol proteases, metallo proteases, exoproteases, endoproteases, proteases involved in the clotting cascade and proteases involved in the classical and/or alternative complement cascade.

In further preferred embodiments of the invention the at least one standard comprises at least one modification-sensitive standard which preferably comprises at least one peptide and/or protein comprising at least one site sensitive to modification, for example at least one phosphorylated amino acid residue and/or at least one recognition sequence for a kinase and/or at least one glycosylated amino acid residue and/or at least one amino acid residue with an attached lipid moiety.

In further embodiments of the methods of the invention the at least one standard comprises at least one standard which is sensitive to proteolytic activity, modification activity and/or peptide loss activity, which activity is typically present in or which activity is suspected to be present in the sample.

In still another embodiment of the invention the at least one standard comprises at least one standard which is derived from the same or similar source as the sample, preferably from serum, plasma, whole blood, urine, liquor cerebrospinalis, thrombocytes, leukocytes, erythrocytes, bacteria, yeasts, fungi, viruses and eukaryotic microorganisms.

In further embodiments of the invention the at least one standard comprises a label, preferably a label selected from the group consisting of fluorescent labels, chromophore labels, luminescent labels, radioisotopic labels, isotopic labels, isobaric labels, enzyme labels, particle labels, labels comprising nucleic acids and tag labels. In these embodiments all standards may comprise the same or different labels or only a part of the at least one standard may comprise the same or different labels.

In further embodiments of the invention the at least one standard comprises a standard which is derived from a species different to the species from which the sample is derived.
Preferably, the at least one standard comprises a standard prepared synthetically or isolated from a natural source. In further preferred embodiments of the invention the at least one standard comprises a standard which is prepared recombinantly.

In further embodiments of the invention the at least one standard comprises a standard which is associated with at least one peptide and/or protein of the sample, preferably by covalent bonding or physical interaction.

In further embodiments the at least one standard comprises a standard which is associated with the surface of the compartment comprising said sample and said at least one standard, preferably by covalent bonding or physical interaction.

In further embodiments of the methods of the invention the sample is derived from whole blood, serum, plasma or urine and is preferably to be analyzed by a method selected from the group consisting of mass spectrometry, SELDI, nuclear magnetic resonance, plasmon resonance, fluorometry, photometry, luminometry, radioactive measurements, enzymatic measurements, microscopy, immunological methods and molecular biological methods.

### Brief description of the drawings

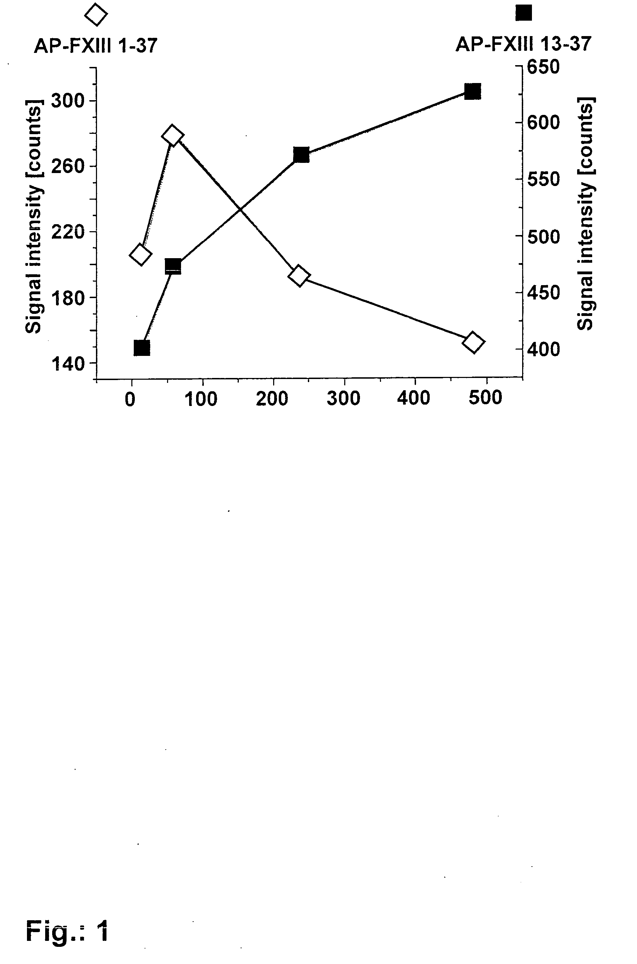

**Figure 1:** Proteolytic degradation of the activation peptide of Factor XIII, amino acids 1-37 (AP-FXIII 1-37) to a fragment corresponding to amino acids 13-37 (AP-FXIII 13-37). Human whole blood was allowed to clot at room temperature for various perionds of time (15 min. to 8 h) and serum samples were collected and immediately frozen and stored at -80°C until analyzed by mass spectrometry. The peptide AP-FXIII 1-37 and its corresponding degradation product AP-FXIII 13-37 were measured. The left y-axis represents the signal intensity for AP-FXIII 1-37, the right y-axis represents the signal intensity for AP-FXIII 13-37 and the x-axis represents the clotting time.

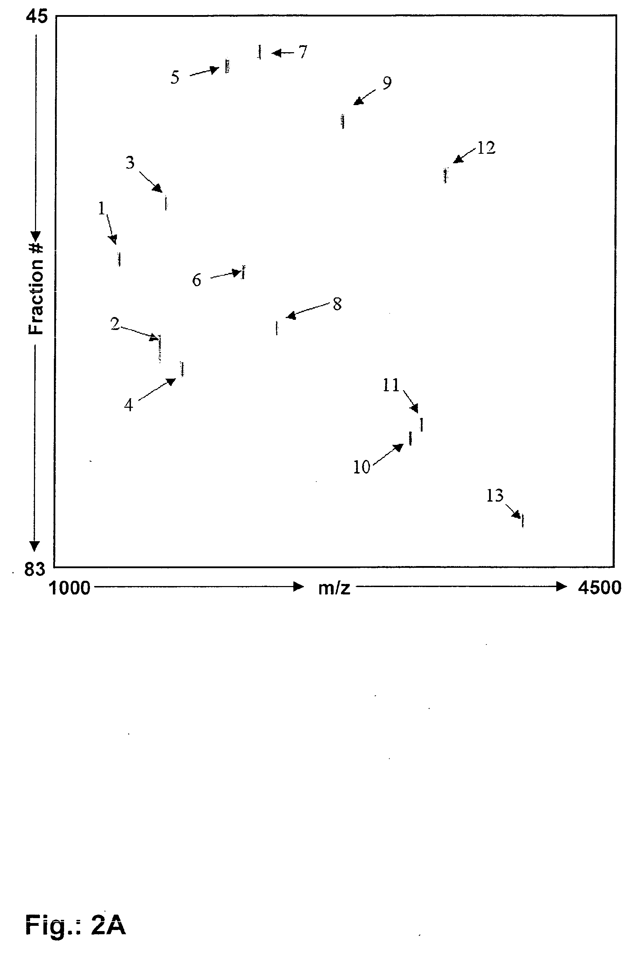

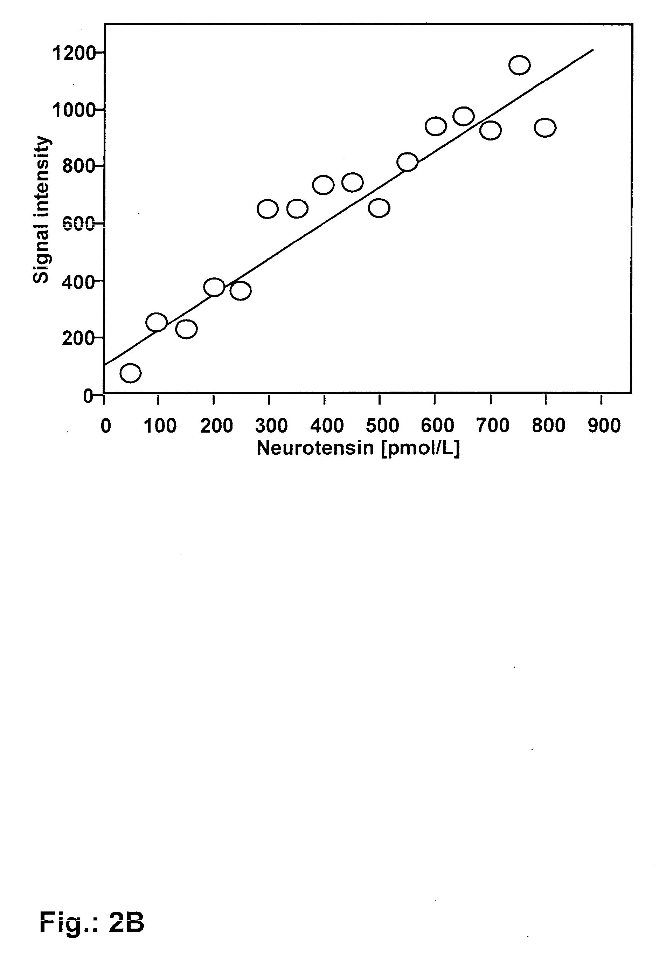

**Figure 2A and 2B**: A mixture of 13 defined peptides, namely 1 = substance P, 2 = somastatin-14, 3 = neurotensin, 4 = renin substrate, 5 = ACTH amino acids 1-17, 6 = endothelin-1 amino acids 19-38, 7 = ACTH amino acids 18-39, 8 = beta-endorphin amino acids 6-31, 9 = ACTH amino acids 1-24, 10 = calcitonin, 11 = insulin B chain, 12 = ACTH amino acids 7-39 and 13 = Pro34 -NPY (neuropeptide Y with proline at position 34) was introduced into plasma samples, subsequently separated by reversed phase chromatography and individual fractions analyzed by mass spectrometry. Mixtures of 50 to 800 pmol of each peptide in the 13-peptide-mix were tested. **Figure 2A** shows the peptide display of the 13 peptides with the y-axis representing the fraction number, the x-axis representing the mass divided through the charge of the ion generated during mass spectrometry (m/z) and the signal intensity depicted by the color intensity of the peptide band in the peptide display. **Figure 2B** shows for the neurotensin peptide (peptide 3) the linear correlation between the signal intensity (y-axis) and the amount of standard peptide neurotensin added to the plasma sample (x-axis).

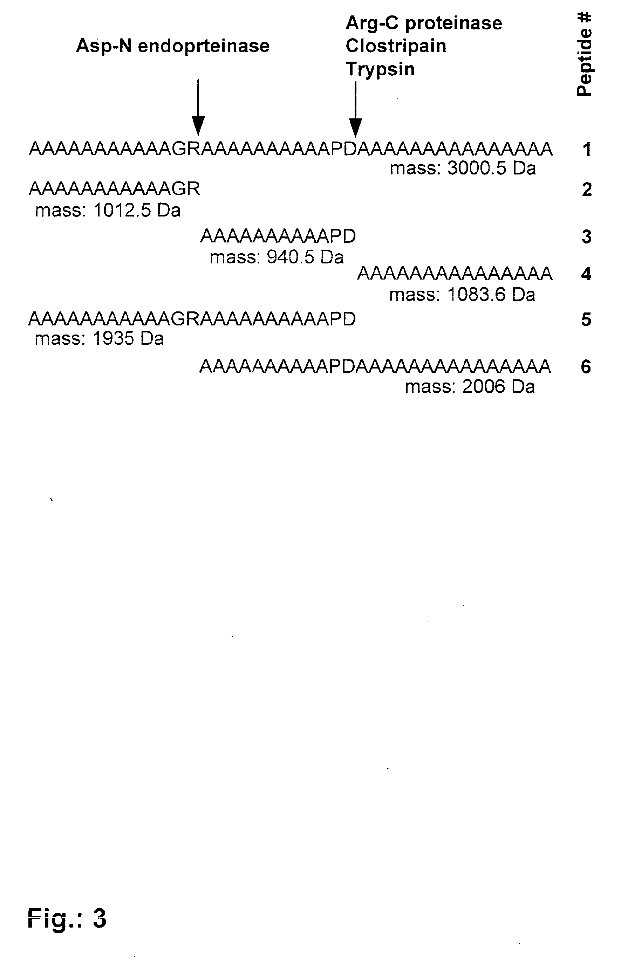

**Figure 3**: Schematic diagram showing the sequence of a designed standard peptide (peptide 1) and five different, potential proteolytic fragments of peptide 1 (peptide 2-6). The peptide sequence was designed to contain distinct protease recognition sites, namely an Asp-N endoproteinase site and a site recognized by Arg-C proteinase, Clostripain and Trypsin. The peptide sequence was designed, so that every fragment has a mass distinct from all the masses of the other peptides.

### Detailed description of the invention

The present invention is directed to the use of standards for determining the status and quality of samples, in particular biological samples comprising peptides and/or proteins. The term "standard" according to the present invention relates to a reference on the basis of which the status and quality of samples can be determined and monitored. In one embodiment, the standards are already present in a compartment and are mixed and/or come into physical contact with the sample, when the sample is filled into the compartment. In a further embodiment, the standards are added to the sample. Thus, the standards and the sample are subjected to exactly the same "environmental factors". Examples of "environmental factors" are among others storage time, storage temperature, temperature changes, freeze-thaw cycles, mechanical mixing, heating or boiling during sample preparation, light exposure, exposure to oxygen or other chemicals such as acids or chaotropic salts, exposure to surfaces, for example the surface of the compartment holding the sample, exposure to enzymes present in the sample, exposure to anticoagulants such as EDTA or citrate or exposure to coagulation-promoting substances optionally present in the compartment, etc. Thereby these standards can be used to indirectly monitor the effects of these "environmental factors" on the substances present in the sample, especially peptides and/or proteins.

The advantage of using these "external" standards instead of peptides naturally present in the sample is, that the standards are known in their identity and quantity at the beginning of the exposure to the "environmental factors" and preferably can be easily and specifically detected. The starting composition and concentration of the standards can be kept constant in different samples regardless whether these samples originated from different individuals, were collected in different laboratories or hospitals, by different technical personal, or were collected over long periods of time, etc. This enables the comparison even between very diverse kinds of samples from various sources.

Another advantage is, that the concentration of the standards is independent from the source of the sample and thereby can be adjusted to concentrations optimal for analysis of alterations of the composition and concentration of the peptides and/or proteins present in the sample to be analyzed.

A further advantage is that the standards do not necessarily have to be peptides which are present in the original sample or can be artificial componds such as non-peptide compounds or compounds comprising peptidic and non-peptidic components comprising a site for proteolytic cleavage and/or modification. This facilitates their easy discrimination from natural peptides present in the sample. For example, the standard may be a self-quenching fluorescence standard which comprises a site for proteolytic cleavage and is labelled with a pair of labels comprised of a fluorescent reporter dye and quencher which interact by energy transfer (FRET) in the uncleaved standard.

Furthermore the standard can be labeled with substances which facilitate the analysis of the effects the "environmental factors" have on them.

In addition it is an advantage, that the standards can be designed in a way that one single standard comprises various properties to monitor "environmental factors". For example one standard could contain several protease recognition sequences, several kinase recognition sequences and in addition could contain several glycosylated amino acid side chains. Such a standard could therefore be used to simultaneously monitor the activity of the corresponding proteases and kinases recognizing the recognition sequences present in the standard and additionally could be used to detect the activity of glycosidases specific for the carbohydrate moieties present in the standard. This enables to monitor various activities possibly present in a sample by using only a limited number of different standards.

It is another advantage of the invention, that the standards could be customized to a certain type of sample. If, for example, HIV positive plasma samples are to be analyzed the standards could be chosen to contain peptides which are substrates of HIV virus specific proteases.

### Samples

According to the invention any sample containing at least one peptide and/or protein is suitable as sample. Preferably the sample contains one, preferably at least 10, preferably at least 50, preferably at least 100, preferably at least 200, preferably at least 600, and preferably at least 1200 different peptides and/or proteins. The term "peptide" refers to compounds consisting of two or more, preferably 3 or more, preferably 4 or more, preferably 6 or more, preferably 8 or more, preferably 10 or more, preferably 13 or more, preferably 16 more, preferably 21 or more amino acids joined covalently by peptide bonds. The term "protein" refers to a polypeptide of more than about 160 amino acids joined covalently by peptide bonds. Peptides mit Mᵣ 20 000 (about 160 amino acid residues) lie on the borderline between polypeptides and proteins. The terms "peptide" and "protein" according to the invention include compounds containing only amino acids, as well as compounds also containing non-amino acid constituents and include compounds containing only peptide bonds and compounds also containing other bonds, e.g. ester, thioether or disulfide.

The peptides can be fragments of all kinds of proteins present in nature including proteins containing postranslational modifications such as phosphate groups, carbohydrate groups or lipid moieties. Also included are peptides which comprise amino acids different from the standard set of 20 amino acids coded by the genetic code. Preferably the sample is whole blood, serum, plasma or urine, serum containing residual blood cells, plasma containing residual blood cells such as thrombocytes, erythrocytes, leukocytes or microorganisms which have infected the individual from which the sample is derived. Preferably the individual is a human, a mammal, a rodent, a primate, a mouse or a rat.

Modifications of peptides or proteins according to the invention may comprise modifications due to postranslational modifications, chemical modifications, enzymatical modifications and modifications due to other mechanisms. Examples of possible modifications include but are not limited to: glycosylation, phosphorylation, sulphatation, pyroglutamate modification, cystein-disulfide bridges, methylation, acetylation, acylation, farnesylation, formylation, geranylgeranylation, biotinylation, stearoylation, palmitylation, lipolyation, C-mannosylation, miristoyliation, amidation, deamidation, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cystein residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides or proteins according to the invention may comprise unusual amino acids, chemically or enzymatically modified amino acids etc. including, but not limited to: alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cystein acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valeine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc. Further examples can be found in databases such as the "Delta Mass" database searchable at the website of the ABRF, the "Association of Biomolecular Resource Facilities": http://www.abrf.org/index.cfm/dm.home?AvgMass=all

### Comparison of samples

In a preferred embodiment the sample is used for comparison with another sample. For meaningful comparisons the samples compared should be derived from the same or from a similar source.

The term "derived from the same or similar source" means that certain substances, materials and/or samples such as two or more different samples or the standard and the sample have the same or a similar origin, and preferably are derived from the same or a related type of tissue or body fluid and/or from individuals of the same class, genus or species and/or from individuals having the same status, such as for example healthy individuals or individuals having the same disease or having been subjected to the same treatment, etc. For example, the standard and the sample are derived from the same or similar source if the sample is serum and the standard is one or more peptides and/or proteins contained in serum.

Examples of "the same or similar sources" among others are samples from the same individual which are taken at different time points or at different day times such as morning, noon, afternoon or night, or before, during or after a medical, experimental or other treatment or before, during or after a disease or before, or before, during or after another condition such as pregnancy, strong physical activity, psychological stress, consumption of food, pharmaceutical substances, alcohol, tobacco products, etc. The samples compared can be taken from the same individual at the same time but from different compartments of the body. For example a blood sample can be taken from a blood vessel before it enters an organ such as liver, lung, kidney or brain and a blood sample can be taken from a blood vessel after it has passed one of these organs. The samples of similar sources to be compared can be from different individuals wherein the samples are taken for example at the same time in the same hospital or the samples are taken from different individuals at different time points and/or at different hospitals or laboratories and/or by different medical or laboratory personal etc. The samples of similar sources to be compared can be stored prior to analysis for various periods of time wherein each sample can be stored for the same time or stored for different periods of time. The samples may or may not have been stored under the same storing conditions for example at room temperature, at 4 °C, at -20 °C, at -70 °C, at -80°C, on dry ice, in liquid nitrogen, etc. The samples may have been thawed once or more often or may not have been thawed prior to analysis, the samples may have been stored under inert gases such as nitrogen or argon or under regular oxygen-containing air. The sample preparation may have been done according to the same protocol or not. For example the clotting time and temperature during the clotting process for different serum samples may be different, the centrifucation time and/or g-force may be different. The urine collected my have been collected in the morning, in the afternoon, before or after a meal or a certain kind of meal such a fat or sugar-rich meal, etc. All of these different kinds of samples of similar sources can be compared and standardized using the method of the invention, as the standards used enable to compare the quality of the sample. By comparison of for example the grade of proteolysis of a standard in different samples it is possible to determine to what extend the sample during collection, processing (for example the clotting process to generate serum) and storage was subjected to proteolysis. By this it is possible to deduce how much the peptides present in a sample, for example a cytokine present in these samples, is affected by proteolysis. So the determination of the degree of the proteolysis of the standard present in a sample enables to estimate the degree of the proteolysis of other analytes such as cytokines present in the same sample. Thus, it can be determined, if different samples are suitable to be used to measure for example cytokines. It can be determined to what extent the measured cytokine levels can be compared or if they are not camparable due to differences in sample quality, or if they have to be mathematically adjusted according to the proteolysis grade determined by use of the standards.

### Quality of samples

The phrase "quality of the sample" according to the invention relates to the integrity of the sample, i.e. whether the sample was subject to alterations of the components contained therein, such as modification and/or degradation and/or change of concentration of the components. The "quality of the sample" may be decisive for the question whether it is suitable for analysis or suitable to be compared with another sample, in particular whether the measured concentration of a biomarker in one sample is comparable to the measured concentration of this biomarker in another sample. This does not necessarily mean that the measured concentration of a biomarker is about the same in different samples but also includes cases where one can trust the measured concentration for a biomarker in one sample as much as one can trust the measured concentration for said biomarker in another sample and the measured concentrations of a biomarker in different samples have the same relative values to each other as they had at a different time point, in particular at the time point when the samples were collected. If, for example, one sample was stored under conditions, which promote proteolytic destruction of a biomarker, and another sample was stored under nondestructive conditions, the measurement of the biomarker in the first sample is too low and not comparable to the measurement of the biomarker in the second sample. The same holds true, if two different samples are used to discover a new biomarker or a new therapeutic molecule by comparing the measured peptide concentrations in the first sample with the measured peptide concentrations in the second sample.

For this purpose the invention makes use of standards which are suitable to monitor the quality of the sample in dependence from such factors as storing conditions, age of sample, etc. If the sample is to be analyzed for peptides or proteins the main factors affecting the peptide composition of the sample are:
- proteases, which digest proteins and/or peptides resulting in peptide and protein fragments
- enzymes such as kinases, phophatases, glycosidases, lipases or nucleases or enzymes catalyzing other chemical reactions, such as oxidation, which alter the structure of peptides and/or proteins
- factors, which result in loss of proteins and/or peptides from the sample, for example by adherence of peptides and/or proteins to sample collection tubes

The standards used according to the invention allow to monitor qualitatively and quantitatively the effects of these sample-modifying factors.

With "protease sensitive standard" is meant a substance which can be cleaved by a protease. The substance is preferably a peptide or protein but can also be different from a peptide or protein, as long as the substance is cleaved by a protease. It is also possible, that the cleavage site present in the standard is different from a peptide bond as long as this cleavage site is cleaved by a protease and thus, the activity of the protease is detectable.

With "modification-sensitive standard" is meant a substance with can be altered by the action of any enzyme, preferably chosen from the group consisting of kinases, phosphatases, glycosidases, lipases and nucleases regardless if these enzymes originate form prokaryotes or eukaryotes, from humans, animals, plants, bacteria or yeasts. A modification-sensitive standard may for example be a peptide and/or protein which can be modified by physical or chemical reactions preferably chosen from oxidation and irradiation with any source of irradiation such as day light, UV-light, infrared light, gamma-rays, beta-rays, alpha-rays, etc. For example methionine, tryptophan or cysteine can be modified by oxidation.

With "standard for monitoring peptide loss" is meant a substance useful for determining the loss, i.e. change in concentration, of peptide and/or protein during collection, processing and/or storage of the sample. For example the concentration of a standard can be decreased by loss of standard due to binding of the standard to the surface of the compartment holding the sample, due to binding of the standard to a blood clot removed during processing of the sample, due to binding of the standard to a membrane used to filter the sample, due to binding of the standard to a protein such as albumin abundantly present in the sample if the abundantly present protein is removed from the sample to facilitate the analysis of the remaining constituents of the sample, etc.

### Methods to analyze the standard and/or the sample

Generally all methods suitable to detect and analyze the standard and/or the sample, in particular peptides and/or proteins of the standard and/or the sample, and/or to detect labels optionally associated with the standard present in the sample are suitable to analyze the standard and/or the sample, in particular by determining qualitatively or quantitatively the standards present in the sample. Such methods include but are not limited to mass spectrometry, MALDI (matrix assisted laser desorption)-mass spectrometry, ESI (electro spray ionisation)-mass spectrometry, TOF (time of flight) mass spectrometry, ICAT (isotope coded affinity tag) mass spectrometry, SELDI (surface enhanced laser desorption) mass spectrometry, plasmon resonance (BIACORE), protein chip arrays, NMR (nuclear magnetic resonance), fluorometry, colorimetry, radiometry, luminometry, FRET (fluorescence resonance energy transfer), etc. The standard can be analyzed prior, after or concomitant with the analysis of the sample.

### Standards:

The present invention envisions the use of at least one standard, preferably 1 to 3, 1 to 5, 1 to 10, 1 to 15, 1 to 20, 1 to 30, and preferably more than 30 standards. If the standards are to be detected by a combination of liquid chromatography and mass spectrometry standards are preferably present in concentrations of e.g. from 1 fmol to 100 mmol, preferably 1 fmol to 100 µmol, preferably from 1 fmol to 100 nmol, preferably from 1 pmol to 100 µmol, preferably from 1 pmol to 100 nmol, preferably from 1 pmol to 100 µmol, preferably form 1 pmol to 100 nmol, preferably from 1 pmol to 1 nmol of each peptide and/or protein of the standard. It is possible, that different peptides and/or proteins within a standard comprising more than one peptide and/or proteins are present in different concentrations. For liquid chromatography online coupled to mass spectrometry the concentrations of the standards are preferably about 10-fold higher, or for SELDI mass spectrometry the concentrations of the standards are preferably about 100- to 1000-fold higher. Other detection methods may require higher or lower concentrations of standards. It is possible to use different standards present in different concentrations in a mixture of standards. This allows to individually adjust the concentration for each standard present in a mixture of standards to an optimal concentration. The concentration of the standards can be determined empirically by testing different concentrations of each standard. For combinations of liquid chromatography and mass spectrometry the molecular mass of the standards can be in the range of 500 Da to 500 kDa, preferably 500 Da to 100 kDa, preferably 500 Da to 50 kDa, preferably 500 Da to 30 kDa, preferably 500 Da to 15 kDa, preferably 500 Da to 10 kDa, preferably 1 kDa to 15 kDa, preferably 1 kDa to 10 kDa, and preferably 1 kDa to 5 kDa. The standards are preferably peptides and/or proteins with or without postranslational modification such as phosphorylations, glycosylations and/or lipid or nucleotide modifications. The standards can be isolated from a natural source, or they can be synthesized using chemical or recombinant technologies, e.g. they can be synthesized by using in-vitro translation systems or by other methods known in the art. The standards can optionally be labeled during synthesis or they can be labeled subsequent to synthesis or subsequent to their isolation from a natural source. The standards can optionally be labeled during the recombinant or in-vitro translation synthesis using for example labeled metabolites. The standards can also be prepared and used without a label. The standards can be purified to homogeneity, namely to a purity between 90 % and 100 %, or they can be substantially purified to a purity between 90 % and 70 %, or they can be purified to purities below 70 % or below 50 % or below 20 % or they can be used without prior purification. The peptides used as standard can comprise sequences and modifications present in nature and they can comprise artificial sequences and modifications not present in nature. The sequence of the peptides and/or proteins used as standards can comprise sequences originating from the same species as the samples tested by use of the standard. Alternatively, the sequence of the peptides and/or proteins used as standard can comprise sequences from a species different to that from which the sample is derived, or the standard can comprise sequences not found in nature, both of which enables a simple discrimination of the standard sequence from the sequences of the peptides and/or proteins present in the sample. It is possible to custom design a sequence of a peptide used as standard so that it is optimally suited for a certain type of sample. This can be done for example by introducing recognition sequences into the standard sequence, which are recognized by enzymes known or expected to be present in the type of samples to be analyzed. For example protease recognition sites of proteases of the clotting cascade can be used to customize peptides as standards for monitoring the quality of plasma samples. This facilitates the easy detection of unwanted proteolytic activities originating form the clotting cascade. Regions of the peptide sequence of a standard can be protected from proteolytic attacks by using protease resistant peptidomimetics leaving only certain protease recognition sequences cleavable by proteases.

### Sample-specific standards

In certain embodiments of the present invention standards are used, which contain recognition sequences of proteases or other enzymes which are specific for or typically present in certain kinds of samples, including body fluids such as serum, plasma, liquor cerebrospinalis or urine and tissues, or specific for or typically present in certain kinds of cells such as blood cells, in particular thrombocytes, erythrocytes or leukocytes. Degradation of standards by these enzymes can be used, for example, as a measure to determine the contamination of a sample, in particular body fluids such as serum, plasma or urine by certain kinds of body fluids, tissues or cells. For example this could indicate the presence of blood in a liquor cerebrospinalis sample or for example, in the case of plasma this could indicate, that due to partial clotting or due to improper plasma preparation thrombocytes have been lysed and released intracellular enzymes, such as proteases into the plasma sample. Examples for enzymes predominantly present in serum and plasma are proteases of the clotting cascade, proteases of the alternative and of the classical complement pathway, etc.

### Standards recognized by microbial enzymes

The standards may also comprise a recognition sequence for enzymes such as proteases, glycosidases, phosphatases, kinases, lipases, nucleases or other enzymes not typical or specific for the species from which the sample originated, but which is typical or specific for a microorganism. Preferably this microorganism is one which can infect individuals of the species, from which the sample originated or more preferably it is a microorganism which is pathogenic for the species, from which the sample originated. Therefore another embodiment of the invention relates to the use of these enzyme activities and their effects on the standards to monitor and control the quality of samples, which samples normally do not contain these enzymes. According to the invention, the term "microorganism" includes bacteria, viruses, yeasts, fungi and eukaryotic microorganisms such as parasites. Examples for such microorganisms are viruses such as rhinoviruses and echoviruses and coxsackieviruses (common cold), influenza viruses A, B and C, parainfluenza viruses 1, 2, 3 and 4, mumps virus, adenoviruses, reoviruses, respiratory syncytial virus, polioviruses, coxsackieviruses, echoviruses, gastroenteritis viruses, rubeola virus (measles), rubella virus (German measles), molluscum contagiosum viruses, human parvovirus B 19, hepatitis viruses type A, B, C, D, E and non-A to E hepatitis viruses, HIV virus 1, 2 or other HIV viruses, cytomegalovirus, Epstein-Barr virus, herpes simplex virus, human herpesvirus type 6, human papillomavirus, varicella-zoster virus, arboviruses such as togaviruses, alphaviruses, flaviviruses, bunyaviruses, phleboviruses and nairovirus, orbivirus, rabies virus, arenaviruses, filoviruses, nora virus, rota virus and hata virus. Examples for such bacteria are Staphylococcus, Streptococcus, Pneumococcus, Neisseria, Erysipelothrix Rhusiopathiae, Listeria, Bacillus anthracis, Bocardia asteroides, Salmonella, Shigella, Escherichia, Klebsiella, Enterobacter, Serratia, Proteus, Morganella, Providencia, Yersinia, Brucella, Francisella tularensis, Vibrio cholerae, Yersinia pestis, Pseudomonas, Campylobacter, Clostridium, Peptococcus, Treponema, Peptostreptococcus, Bacteroides, Prevotella, Fusobacterium, Leptospira, Borrelia burgdorferi, Streptobacillus, Spirillum, Nocardia, Rickettsia, Bartonella, Chlamydia, and Mycobacterium. Examples for such fungi are Histoplasma capsulatum, Coccidioides immitis, Blastomyces dermatitidis, Paracoccidioides brasiliensis, Sporothrix schenckii, Cryptococcus neoformans, Candida, Aspergillus, Rhizopus, Rhizomucor, Absidia, Basidiobolus, Bipolaris, Cladophialophora, Cladosporium, Drechslera, Exophiala, Fonsecaea, Phialophora, Xylohypha, Ochroconis, Phinocladiella, Scolecobasidium, Wangiella, Trichosporon, Blastoschizomyces, Malassezia, Penicillium and Actinomyces. Examples for such parasites are Plasmodium, Trypanosoma, Leishmania, Naegleria, Hartmannella, Acanthamoeba, Giardia, Strongyloides, Entamoeba, Shistosoma, Cryptosporidium, Isospora, Cyclospora, E. histolytica, Trichuris, Ascaris, Hookworms, Strongyloides, Tapeworms, Flukes, Enterobius, Paragonimus, Echinococcus, Onchocera, Trichomonas and Schistosoma haematobium.

### Peptidomimetics

Peptides used as standards can be prepared or synthesized to represent partially or completely peptidomimetics. This allows to design peptides with more defined properties. For example D-amino acids could be used to prevent proteolysis of all peptide bonds, except for example defined protease recognition sequences, which were introduced into the standard to monitor the activity of certain proteases within the sample and thereby to monitor the sample quality. "Peptidomimetics" according to the invention are structures, which mimic the function of amino acids or amino acid sequences. "Peptidomimetics" or "mimetics of peptides" often have additional properties such as increased resistance to proteolysis. Peptidomimetics can replace some or all peptide bonds by other kinds of covalent bonds which can connect amino acids. Peptidomimetics among others can comprise modifications of amino acids such as alpha-C alkylation, alpha-N alkylation, etc, dipeptide analogues (two amino acid side chains which are connected by covalent bonds) or modifications of the peptide backbone, especially change from L- to D-amino acids, inverse N- to C-sequence, amide bond isosteres, etc. Further examples of peptidomimetics are spiegelmers® (NOXXON Parma AG, Berlin, Germany) or beta amino acids.

### Labels

The invention envisions the use of standards, which comprise at least one label.

The term "label", as used herein, refers to any moiety that functions to: (i) provide a detectable signal; (ii) interact with a second label to modify the detectable signal provided by the first or second label, e.g. FRET; (iii) affect mobility, e.g. electrophoretic mobility, by charge, hydrophobicity, shape, or other physical parameters, or (iv) provide a capture moiety, e.g., affinity, antibody/antigen, or ionic complexation.

Suitable as label are all kinds of structures, such as isotopic labels, isobar labels, fluorescent labels, radioactive labels, luminescent labels, enzyme labels, toxin labels, dye labels, metal particles as labels, magnetic particles as labels, polymer particles as labels, biotin or other organic molecules as labels, etc.

For example isotopes with molecular weights different to their natural molecular weight such as oxygen-18, nitrogen-15, deuterium, tritium, carbon-14, sulfur-35, phosphorus-32 or phosphorus-33 etc. can be used to prepare isotope-labeled standards. The isotopes can be incorporated directly into the standards by using isotope-labeled amino acids for synthesis of the standards. Furthermore, these isotopes can be incorporated into standards by growing of cells in the presence of isotope-labeled metabolites and isolating the labeled standards from these cells or cell culture supernatants. The isotopes can also be incorporated into standards by other methods such as in vitro translation using isotope-labeled metabolites. Isotope-labeled metabolites can be amino acids, carbohydrates, fatty acids, inorganic salts such as phosphate, sulfate, etc. or other isotope-labeled substances. These isotope-labeled standards are especially suitable for measuring methods using mass spectrometric methods. Isotope-labeled peptides are especially suitable if the standards are used in methods employing mass spectrometric methods.

Another kind of label especially suitable for mass spectrometric detection methods are isobaric labels. Different kinds of such labels have the same molecular weight, as long as these labels are intact and have not been fragmented in a mass spectrometer. This has the advantage, that mixtures of molecules labeled with different isobar labels can more conveniently be analyzed using mass spectrometric methods, as only a limited number of mass differences is present prior to analysis of the sample. Fragmentation of the isobar labels is done to determine the identity of the label, thereby identifying the identity of the molecule labeled with the isobar label. Preferably these labels are constructed in a way that upon fragmentation of the label in a mass spectrometer the fragments generated have masses, which masses usually are not obtained by fragmentation of peptides or proteins. Examples of such masses are masses between 114 and 120 Da. Genreally isobaric labels should be selected, which generate masses, which are not generated in the same kind of samples, analyzed by the same mass spcetrometric method in the absence said isobaric labels.

Alternatively, to directly using labeled amino acids in peptide synthesis, peptides and proteins generally can be labeled using amine-reactive reagents such as isothiocyanates, succinimidyl esters and carboxylic acids, sulfonyl chlorides, aldehydes, arylating reagents, etc. or thiol- reactive reagents such as iodoacetamides, maleimides, alkylhalides and arylating agents. Furthermore alcoholes such as those present in serine, threonine or in carbohydrate moieties can be labeled by oxidizing with periodate to yield aldehydes that can be subsequently modified with a variety of amine or hydrazine derivatives. Alcoholes from tyrosine sometimes can be reacted with sulfonyl chlorides or iodoacetamides. Alcoholes in carbohydrate moieties furthermore can be labeled by reaction with dichlorotriazines. N-methylisatoic anhydride will convert ribonucleotides and other carbohydrate moieties to fluorescent esters with excitation/emission maxima of about 350/466 nm. Examples of fluorescent labels are fluorophores such as Alexa Fluor® 350, 405, 430, 488, 500, 514, 532, 546, 555, 568, 594, 610, 633, 635, 647, 660, 680, 700 and Alexa Fluor® 750, Aminomethylcoumarin (AMCA), Methoxy coumarin acetic acid, Bimane, BODIPY 493/503, 530/550, 558/568, 564/570, 576/589, 581/591, 630/650 and BODIPY 650/665, BODIPY FL, BODIPY TR; BODIPY TMR, Cascade Blue dye, Cascade Yellow dye, cyanine dyes such as Cy3, Cy5, Cy5.5, Cy7, Dansyl, Dapoxyl dye, Dialkylaminocoumarin, Eosin, Erythrosin, Fluorescein (FITC), Fluorescein-EX, 2',4',5',7'-Tetrabromosulfonefluorescein, Naphthofluorescein, 2',7'-Dichloro-fluorescein, 4',5'-Dichloro-2',7'-dimethoxy-fluorescein, 6-Carboxy-2',4,4',5',7,7'-hexachlorofluorescein, succinimidyl ester (6-HEX, SE), 5-carboxyfluoresceine (5FAM), 5,6-carboxyfluoresceine (5(6)FAM), hydroxycoumarin, Malachite green, Marina Blue dye, methoxycoumarin, 7-nitro-4-benzofurazanyl (NBD), Oregon Green® 488, Oregon Green® 514, Pacific Blue, Pacific Blue dye, PyMPO, Pyrene, QSY 7 , QSY 9, QSY 21, QSY 35, Rhodamine 6G, Rhodamine Green, Rhodamine Green dye, Rhodamine Red dye, Rhodamine Red, Rhodamine Red-X (RRX), X-rhodamine, Tetramethyl-rhodamine (TMR, TRITC), Lissamine rhodamine B, Texas Red, Texas Red dye, Texas Red-X. Most of these fluorophores are available from Molecular Probes, Eugene, OR, USA, and many are also available as kits intended for protein or peptide labeling. Furthermore fluorescent proteins or fragments or derivatives thereof such as green fluorescent protein, red fluorescent protein, blue fluorescent protein, renilla fluorescent protein, etc. can be used as labels. Examples of radioactive isotopes suitable as labels are phosphorous-32 or - 33, sulphur-35, iodine-125, tritium, carbon-14, calcium-45, chromium-51 and all other known radioisotopes which can be coupled to nucleic acids and/or peptides or proteins. It is also possible to use matched pairs of fluorescent labels, which are suitable for fluorescence resonance energy transfer (FRET).

Examples of enzyme labels are horsereddish-peroxidase, alkaline phosphatase, luciferase, galactosidase, etc. Suitable toxin labels are all kinds of toxins such as microbial toxins, toxic peptides or toxic small organic molecules, synthetic toxins or toxins used for medical purposes. An example of suitable dye labels is digoxigenin. Examples of particles as labels are particles of various particle diameters, preferably between 0.1 to 100 µm. Metal particles can be made from various substances, preferably heavy elements such as gold, silver, platinum or other suitable metals or alloys or mixtures thereof. Examples of magnetic particles are preferably made from all kinds of magnetic or magnetizeable materials including iron, preferably iron oxides such as Fe₃O, Fe₂O₃, Fe₃O₄, etc. Examples of polymer particles are particles made from a polymer such as polyacryl amide, agarose, polypropylene, polystyrene, polytetrafluoroethylene, fluorescent polystyrene microspheres, etc. Various other labels, such as biotin, chitin, maltose or glutathione etc. as known in the art, can also be used.

Furthermore labels comprising certain additional nucleic acid or amino acid sequences subsequently termed label-sequences can be used. Label-sequences comprising nucleic acid sequences for example can be used for detection of the labeled substance for example by hybridization with a probe specific for the label-sequence or by detection of the label-sequence by polymerase chain reaction, preferably by quantitative polymerase chain reaction, preferably by real-time polymerase chain reaction, etc. Examples of label-sequences comprising amino acid sequences are his-tags, flag-tags, myc-tags or whole proteins or fragments thereof such as antibodies, glutathione S-transferase, streptavidin, chitin-binding protein, maltose binding protein, various lectins, etc. These labels can be detected by use of antibodies or by use of ligands binding to these labels such as protein A, protein G, protein Y, protein A/G, glutathione, biotin, chitin, maltose or other sugars etc. Label-sequences in general can also be used to capture the labeled standard by a binding agent specific for said label-sequence. Subsequently or during the capture process the standard can be quantitated.

Labels can be at the N- or C-terminus or can be present internal in the amino acid sequence of a peptide and/or protein.

### Properties of the compartment

The surface of the compartment which holds the sample and the standard can be treated to prevent or reduce binding of proteins and/or peptides. This can be achieved by coating the surface with proteins, nanoparticles, hydrophobic substances such as silicone, etc. Also the use of certain materials such as fluorocarbon resin (Teflon®), which prevent protein or peptide binding to the compartment, can reduce peptide or protein loss from the sample, thereby preventing impairment of the quality of the sample.

In general the compartment can be manufactured from all suitable materials such as plastic, glass or metal. Preferably the compartment is made from plastic material such as polyethylene, polypropylene, polybutadiene, polystyrol, polyvinylchloride, polytetrafluorethene (Teflon®), polymethacrylic acid ester, polyester, formaldehyde resins, polycarbonate, polyethylene terephthalate (PET), polybutylene terephthalate (PBT), etc. as plastic is less prone to break resulting in shards which my injury for example laboratory personal using the compartment for holding the samples and standards.

The compartment furthermore can contain means for separating different phases of the sample, preferably to separate a blood clot from serum or blood cells, thrombocytes and other particles from plasma. Suitable for separation are membranes which allow only the liquid components of the sample to pass the membrane or gels which float, as a result of their specific mass between the different phases, preferably between the cellular phase and the plasma phase. Preferably, a thioxotropic gel can be used for this (US 5,906,744). Also a pellet or disc, which floats between two phases, for example a blood cell phase and a plasma phase, and which increases in size as it comes into contact with water and thereby seals the compartment to form two separate sub-compartments can be used (US 5,736,033).

The compartment itself can have various shapes, as long it is suitable to collect and/or store the sample. Suitable compartments among others are tubes, closed system blood collection devices, collection bags, syringes, pre-filled syringes, catheters, microtiter plates, multi-well collection devices, flasks, spinner flasks, roller bottles, vials, pipettes, pipette tips, capillaries, needles and other compartments suitable for holding a sample.

A special type of compartment regarding the invention is a surface which is suitable to store and/or analyze a sample. Examples for such surfaces are the surface of chips such as protein chips, SELDI chips (SELDI =surface enhanced laser desorption ionisation, Ciphergen Biosystems, Inc., Fremont, CA, USA), surface plasmon resonance chips (BIACORE®, Biacore International SA, Freiburg, Germany), targets used for mass spectrometry (= plates usually made from metal or plastic onto which drops of samples are applied and which plates are inserted into a mass spectrometer to analyze the sample). In general these kinds of chip surfaces can be made of various materials including plastic such as fluorocarbon resin (used for targets), metals such as gold (used for plasmonresonance and SELDI), glass (used for protein chips), coated surfaces with "chromatography resin-like" properties such as protein chips with anion or cation exchange, reversed phase, metal affinity binding properties or the ability to covalently connect molecules at the chip surface. Protein chips modified in such a way are for example sold by Ciphergen Biosystems, Inc. Fremont, CA, USA.

### Substances to improve sample quality

Furthermore the compartment may contain additional substances, which may improve the sample quality including substances which inhibit microbial growth such as antibiotics, ethylmercury (thimerosal), sodium azide, etc., substances which inhibit foaming of the samples such as ethylene oxide/propylene oxide block copolymers (pluronic®), substances which inhibit coagulation or which promote coagulation, proteases such as inhibitors of serine-, threonine-, cysteine-, aspartic-, glutamic-, metallo-, and other proteases.

### Anticoagulants and coagulation-promoting substances

The sample can be any kind of sample containing peptides and/or proteins. Preferably the sample is a biological liquid commonly collected such as blood plasma, blood serum, whole blood and urine. Plasma can be obtained by adding anticoagulants directly upon drawing of the blood sample. Suitable anticoagulants among others are EDTA (ethylenediaminetetraacetic acid), citrate or oxalate salts, heparin, antithrombin, hirudin, etc. or more general substances which chelate divalent cations such as calcium, substances which inhibit enzymes involved in the clotting cascade, etc. Serum can be obtained by incubation of a blood sample at room temperature, at 4 °C or at other temperatures for appropriate time intervals to allow clotting as known in the art. Serum can also be obtained by exposure of a blood or plasma sample to agents, which promote clotting such as thrombin and other substances or enzymes involved in or supporting or activating the clotting cascade. Also the presence of particles such as glass beads, increasing the surface in contact with the blood, are suitable to promote clotting. In a preferred embodiment of the invention the compartment used to hold the sample and the standard comprises a compartment which in addition to standards contains anticoagulants or coagulation-promoting substances.

### Protease inhibitors

Examples of protease inhibitors are EDTA, EGTA, PMSF (phenyl methyl sulfonyl fluoride), EGTA, benzamidine, Prefabloc SC, TLCK (1-chloro-3-tosylamido-7-amino-2-heptanone), TPCK (1-chloro-3-tosylamido-4-phenyl-2-butanone), Di-isopropylfluorophosphate (DPF), 3,4-dichloroisocoumarin (DCI), Phosphoramidon (N-alpha-L-rhamnopyranosyloxy(hydroxyphosphinyl)-L-Leucyl-L-Tryptophan), 1,10-Phenanthroline, Pepstatin (Isovaleryl-Val-Val-AHMHA-Ala-AHMHA where AHMHA=(3S, 4S)-4-amino-3-hydroxy-6-methyl-heptanoic acid, Leupeptin (Acetyl-leucyl-leucyl-arginal), Elastatinal (Leu-(Cap)-Gln-Ala-al, N-[(S)-1carboxy-isopentyl)-carbamoyl-alpha-(2-iminohexahydro-4(S)-pyrimidyl]-L-glycyl-L-glutaminyl-L-alaninal), E-64 (L-trans-epoxysuccinyl-leucylamide-(4-guanido)-butane, chymostatin (Phe-(Cap)-Leu-Phe-al N-[(S)-1-carboxy-isopentyl)-carbamoyl-alpha-(2-iminohexahydro-4(S)-pyrimidyl]-L-glycyl-L-phenylalaninal, bestatin ([(2S, 2R)-3-Amino-2-hydroxy-4-Phenylbutanoyl]-L-Leucine), APMSF ((4-AmidinoPhenyl)-Methane-Sulfonyl Fluoride), antipain ([(S)-1-Carboxy-2-Phenyl]-carbamoyl-Arg-Val-arginal), amastatin ([(2S, 2R)]-3-Amino-2-hydroxy-5-methylhexanoyl]-Val-Val-Asp-OH). These inhibitors are commercially available form vendors such as Sigma-Aldrich Chemie GmbH, Munich, Germany.

### Phosphatase inhibitors

Examples of phosphatase inhibitors are sodium fluoride, sodium molybdate, sodium orthovanadate, sodium tartrate dihydrate, okadaic acid, dephostatin, 3-hexadecanoyl-5-hydroxymethyl-tetronic acid, (-)-p-bromotetramisole bxalat, cantharidin, microcystin-LR, alpha-bromo-4-hydroxyacetophenone 4-hydroxyphenacyl Br, alpha-bromo-4-methoxyacetophenone 4-methoxyphenacyl Br, alpha-bromo-4-(carboxymethoxy)acetophenone 4-(carboxymethoxy)phenacyl Br, 3-hexadecanoyl-5-hydroxymethyl-tetronic acid, imidazole, etc. These inhibitors are for example commercially available from Calbiochem, San Diego, CA, USA or Biosource International, Camarillo, CA, USA

### Kinase inhibitors

Examples of kinase inhibitors are N-[2-(methylamino)ethyl]-5-isoquinolinesulfonamide dihydrochloride, N-[2-(p-bromocinnamylamino) ethyl]-5-isoquinoline sulfonamide, 1-(5-isoquinolinesulfonyl)-piperazine dihydrochloride, N-(2'-guanidinoethyl)-5 isoquinolinesulfonamide dihydrochloride, 1-(5-isoquinolinesulfonyl)-1H-hexahydro-1,4-diazepine, 1-[N,O-bis-(5-isoquinolinesulfonyl) -N-methyl-L-tyrosyl]-4-phenyl-piperazine, Aloisine (7-n-butyl-6-(4-methoxyphenyl)[5 H]pyrrolo[2,3-b]pyrazine), Aloisine A (7-n-butyl-6-(4-methoxyphenyl)[5 H]pyrrolo[2,3-b]pyrazine), edelfosine (2-O-methyl-1-O-octadecyl-rac-glycero-3-phosphocholine), 4-(4-fluorophenyl)-2-(4-methylsulfinyl phenyl)-5-(4-pyridyl) 1H-imidazole, 4-amino-5-(4-methylphenyl)-7-(t- butyl)pyrazolo[3,4-d]pyrimidine and 4-amino-5-(4-chlorophenyl)-7-(t- butyl)pyrazolo[3,4,d]pyrimidine which for example are commercially available from Biaffin GmbH & Co KG, Kassel, Germany.

### Glycosidase inhibitors

Examples of glycosidase inhibitors are Australine hydrochloride, Castanospermine, 6-acetamido-6-deoxy-castanospermine, Deoxynojirimycin (DNJ), Deoxyfuconojirimycin hydrochloride (DFJ), Deoxygalactonojirimycin hydrochloride (DGJ), Deoxymannojirimycin hydrochloride (DMJ), Deoxymannojirimycin (DMJ), 2,5-dideoxy-2,5-imino-D-mannitol, 1,4-dideoxy-1,4-imino-D-mannitol hydrochloride, 1,4-dideoxy-1,4-imino-D-xylitol hydrochloride, (3R,4R,5R,6R)-3,4,5,6-tetrahydroxyazepane hydrochloride, (3S,4S,5S,6S)-3,4,5,6-tetrahydroxyazepane hydrochloride, 1,5-dideoxy-1,5-imino-xylitol and Kifunensine which are for example commercially available from Industrial Research, Auckland, New Zealand.

### Chaotropic salts

Another class of substances, which can improve the quality of a sample regarding peptide composition are chaotropic salts such as urea, guanidinium hydrochloride, sodium thiocyanate, calcium chloride, barium chloride, sodium sulphate, ammonium sulphate, etc. These substances denature very rapidly all kinds of enzymes and prevent enzymatic action on the peptides present in the sample thereby conserving the original composition of the sample at the time point when the sample was taken. Consequently in addition to standards chaotropic salts may be present in the compartment used to hold the sample.

Preferably all of the previously named inhibitors optionally used to improve the quality of the sample should not be peptides or proteins but small organic molecules or very small peptides, preferably smaller than 1000 Dalton, to avoid interference with analysis of the sample. This is of particular importance, as inhibitors often need to be used in large amounts to be effective.

### Examples

The following examples are intended to illustrate the invention, however they are not meant to limit the scope of the invention in any way.

### Example 1: Preparation of serum and plasma

Healthy adult men were enrolled in this study after they provided written informed consent and the local Ethics Committee at the Hannover Medical School approved the protocol. The blood samples were collected from the cubical vein into blood collection tubes (EDTA-plasma: 9 ml S-Monovette containing potassium EDTA; citrate-plasma: 9 ml S-Monovette containing 0.106 mol/L citrate solution; serum: 9 ml S-Monovette with clot activator, Sarstedt, Nümbrecht, Germany). If the samples were stored and analyzed with standards the standards were added to the blood immediately after drawing of the blood or the standards were added to the samples immediately after the samples were thawed. It should be noted, that these experiment were not done with standards already present in the blood drawing devices (S-Monovette), but that the idea of the invention is to provide such blood drawing devices or other sample collecting or storing compartments, which contain already the standard or into which the standards are given prior to or after filling the compartments with sample. Immediately after withdrawal, plasma was obtained by centrifugation for 10 min. at 2000 x g at room temperature. Centrifugation at low temperatures, such as 4°C should be avoided, as low temperatures activate thrombocytes to, among others, release proteins and peptides into the plasma, which negatively affects the sample quality for analysis of the peptides or proteins present in the plasma sample. Plasma samples of 2 ml volume were transferred into 2 ml tubes fitted with a cellulose acetate filter unit with 0.2 µm pore size and 5 cm² filtration area (Satorius Minisart®, Sarstedt, Nümbrecht, Germany). Plasma samples were transferred to a -80 °C freezer until further analysis. Serum samples were obtained after coagulation of blood for 1 h at room temperature. The collection tube was centrifuged for 10 min. at 4°C at 2000 x g. Serum samples were transferred to a -80 °C freezer until further analysis. For analysis of the activation peptide of factor XIII, amino acids 1-37 (AP-FXIII 1-37) and of the corresponding fragment of the peptide (AP-FXIII 13-37) serum samples were incubated for 15 min., 1 h, 4 h and 8 h at room temperature (figure 1) and subsequently analyzed by liquid chromatography and mass spectrometry (examples 2 and 3).

### Example 2: Liquid chromatography of the samples

The separation method carried out was a reverse phase chromatography. Various RP chromatography resins and eluants are equally suitable. The separation of peptides and/or proteins using a C18 reverse phase chromatography column with a size of 4 mm x 250 mm was done as described below. Mobile phases of the following compositions were used: mobile phase A: 0.06 % (v/v) trifluoroacetic acid, mobile phase B: 0.05 % (v/v) trifluoroacetic acid, 80 % (v/v) acetonitrile. The chromatography took place at 33°C using an HP ChemStation 1100 supplied by Agilent Technologies with a micro flow cell supplied by Agilent Technologies.
The samples were diluted with 0.05% (v/v) trifluoroacetic acid, the pH was adjusted to 2-3, the sample was centrifuged at 18 000 x g for 10 minutes and finally 750 µl of serum or plasma equivalent prepared in this way were loaded onto the chromatography column representing an equivalent of 15 µl plasma or serum. The chromatography conditions were as follows: 5 % mobile phase B at time 0 min, from time 1 to 45 min continuous increase in the mobile phase B concentration to 50 %, from time 45 to 49 min continuous increase in the mobile phase B concentration to 100 % and subsequently up to time 53 min constant 100 % buffer B. Collection of 96 fractions each of 0.5 ml starts 10 minutes after the start of the chromatography.

### Example 3: Mass spectrometry of samples

For mass spectrometric analysis, typical positive ion spectra of peptides were produced in a MALDI-TOF mass spectrometer (matrix-assisted laser desorption ionization). Suitable MALDI-TOF mass spectrometers are manufactured by PerSeptive Biosystems Framingham (Voyager-DE, Voyager-DE PRO or Voyager-DE STR) or by Bruker Daltonik Bremen (BIFLEX). The samples are prepared by mixing them with a matrix substance which typically consists of an organic acid. Typical matrix substances suitable for peptides are 3,5-dimethoxy-4-hydroxycinnamic acid, α-cyano-4-hydroxycinnamic acid and 2,5-dihydroxybenzoic acid. A lyophilized equivalent obtained by reverse phase chromatography and corresponding to 15 µl plasma or serum is used to measure the peptides and/or proteins and/or standards. The chromatographed sample is dissolved in 15 µl of a matrix solution. This matrix solution contains, for example, 10 g/l α-cyano-4-hydroxycinnamic acid and 10 g/l L(-)fucose dissolved in a solvent mixture consisting of acetonitrile, water, trifluoroacetic acid and acetone in the ratio 49:49:1:1 by volume. 0.3 µl of this solution is transferred to a MALDI carrier plate, and the dried sample is analyzed in a Voyager-DE STR MALDI mass spectrometer from PerSeptive Biosystems. The measurement takes place in linear mode with delayed extractionTM. The MALDI-TOF mass spectrometer can be employed to quantify peptides such as, for example, the standard peptides of the invention if these peptides are present in a concentration which is within the dynamic measurement range of the mass spectrometer, thus avoiding detector saturation. There is a specific ratio between measured signal and concentration for each peptide, which means that the MALDI mass spectrometry can preferably be used for the relative quantification of peptides (figure 2B). It is possible to measure the signal intensities of of the standards and of peptides originating from the sample.

### Example 4: Peptide identification

The standard consisting of peptides can be identified for example by using nanoSpray-MS/MS. This entails a standard peptide ion being selected in the mass spectrometer on the basis of its specific m/z (mass/charge) value in a manner known to the skilled worker. This selected ion is then fragmented by supplying collision energy with an collision gas, e.g. helium or nitrogen, and the resulting fragments of the standard peptide are detected in the mass spectrometer in an integrated analysis unit, and corresponding m/z values are determined (principle of tandem mass spectrometry). The fragmentation behaviour of peptides makes unambiguous identification of the peptides possible. In this specific case, the mass-spectrometric analysis took place with a Quadrupol-TOF Instrument, QStar-Pulsar model from Applied Biosystems-Sciex, USA.

### Example 5: Data analysis

Subsequently to fractionation as described in example 1, each fraction is individually analyzed by MALDI mass spectrometry as described in example 3 resulting in 96 mass spectra for each sample. These 96 mass spectra are electronically combined to a so called peptide display. The x-axis of these peptide displays depicts the molecular mass, the y-axis depicts the fraction number and the colour intensity represents the mass spectrometric signal intensity. The data of peptide displays may be pre-processed by adjusting for background noise and outliers may be removed from the analysis. Differences between peptide displays are calculated by subtracting peptide displays from each other electronically. Detection of different concentrations of peptides is done by comparison of the mass spectrometric data (signal intensities) from serum samples incubated for different time intervals (figure 1) or by comparison of plasma samples spiked with different concentrations of the 13-peptide-mix (figure 2B). To distinguish the 13 standard peptides from the thousands of signal originating from other peptides present in the plasma sample a correlation analysis was done. Peptide displays obtained with plasma samples into which different concentrations of the standard peptide mix were spiked were compared by correlation analysis using a correlation coefficient of r=0.8. This high correlation value r results only, because the concentrations of the standard peptides alter so uniformly from sample to sample (as different concentrations were added to different serum samples).

### Example 6: Design of custom standard peptides

Figure 3 shows the sequence of a standard peptide designed to contain 2 distinct protease restrictions sites (Peptide 1 in figure 3, Seq.-ID 1). The peptide of Seq.-ID 1 was designed using the knowledge of the recognition sites of a set of proteases which is shown in table 1. The following proteases were considered for the calculation of the fragments proposedly generated from the designed peptide (Peptide 1 in figure 3): Arg-C proteinase, Asp-N endopeptidase, Caspase1, Caspase2, Caspase3, Caspase4, Caspase5, Caspase6, Caspase7, Caspase8, Caspase9, Caspase10, Chymotrypsin-high specificity (C-term to [FYW], not before P), Chymotrypsin-low specificity (C-term to [FYWML], not before P), Clostripain, Enterokinase, GranzymeB, Pepsin, Proline-endopeptidase, Staphylococcal, peptidase I, Factor Xa, Glutamylendopeptidase, LysC, Thrombin, Trypsin. The result of using the information of table 1 and the sequence of the peptide of Seq.-ID 1 is, that the proteases theoretically cut the peptide between amino acids 13 and 14 ( Asp-N endoproteinase) and between amino acids 25 and 26 (Arg-C proteinase and/or Clostripain and/or Trypsin). If both protease recognition sites cut the same molecule of peptide 1, this results in three different, distinct fragments of Seq.-ID 1 represented by peptide 2 (= Seq.-ID 2), peptide 3 (Seq.-ID 3) and peptide 4 (Seq.-ID 4). If a molecule of peptide 1 is cut only at one of the two potential protease recognition sites there can be generated two other fragments of peptide 1, namely peptide 5 (Seq.-ID 5) and peptide 6 (Seq.-ID 6). Using the software GPMAW v. 4.02 the monoisotopic molecular masses of all 5 fragments of peptide 1 and of peptide 1 itself were calculated. These masses are 3000.5 Da for peptide 1, 1012.5 Da for peptide 2, 940.5 Da for peptide 3, 1083.6 Da for peptide 4, 1935 Da for peptide 5 and 2006 Da for peptide 6. Consequently if the designed peptide 1 is used as standard in a sample containing all of the above listed proteases only these 6 distinct masses could be detected for the standard peptide 1. In addition, this example shows, that designed standards can comprise multiple enzyme recognitions sites, not only protease sites but also other enzyme recognition sites, which result in modification of the standard and which result in a distinct and predictable mass change of the standard. The calculation done in this example can also be done on the SwissProt webpage: http://au.expasy.org/tools/peptidecutter/

**Table 1: Protease recognition sequences of the proteases used in Example 6.**

| **Enzyme name** | **P4** | **P3** | **P2** | **P1** | **P1'** | **P2'** |
|---|---|---|---|---|---|---|
| Arg-C proteinase | - | - | - | R | - | - |
| Asp-N endopeptidase | - | - | - | D | - | - |
| Caspase 1 | W or H | E | H | D | not P, E, D, Q, K or R | - |
| Caspase 2 | D | V | A | D | not P, E, D, Q, K or R | - |
| Caspase 3 | D | M | Q | D | not P, E, D, Q, K or R | - |
| Caspase 4 | L | E | V | D | not P, E, D, Q, K or R | - |
| Caspase 5 | L or W | E | H | D | - | - |
| Caspase 6 | V | E | H or I | D | not P, E, D, Q, K or R | - |
| Caspase 7 | D | E | V | D | not P, E, D, Q, K or R | - |
| Caspase 8 | I or L | E | T | D | not P, E, D, Q, K or R | - |
| Caspase 9 | L | E | H | D | - | - |
| Caspase 10 | I | E | A | D | - | - |
| Chymotrypsin-high specificity (C-term to [FYW], not before P) | - | - | - | F or Y | not P | - |
| | - | - | - | W | not M or P | - |
| Chymotrypsin-low specificity (C-term to [FYWML], not before P) | - | - | - | F,L or Y | not P | - |
| | - | - | - | W | not M or P | - |
| | - | - | - | M | not P or Y | - |
| | - | - | - | H | not D,M,P or T | - |
| Closiripain | - | - | - | R | - | - |
| Enterokinase | D or N | D or N | D or N | K | - | - |
| Factor Xa | A,F,G,I,L, T,V or M | D or E | G | R | - | - |
| Glutamyl | - | - | - | E | - | |
| endopeptidase | | | | | | |
| GranzymeB | I | E | P | D | - | - |
| Hydroxylamine | - | - | - | N | G | - |
| LysC | - | - | - | K | - | - |
| Pepsin (pH1.3) | - | not H,K, or R | not P | not R | F,L,W or Y | not P |
| | - | not H,K, or R | not P | F,L,W or Y | - | not P |
| Pepsin (pH>2) | - | not H,K or R | not P | not R | F or L | not P |
| | - | not H,K or R | not P | F or L | - | not P |
| Proline-endopeptidase | - | - | H,K or R | P | not P | - |
| Staphylococcal peptidase I | - | - | not E | E | - | - |
| Thrombin | - | - | G | R | G | - |
| Trypsin | - | - | - | K or R | not P | - |
| (please note | - | - | W | K | P | - |
| exceptions) | - | - | M | R | P | - |

| The above cleavage rules for Trypsin do not apply, i.e. no cleavage occurs, with the following compositions of the cleavage sites: | | | | | | |
|---|---|---|---|---|---|---|
| | **P4** | **P3** | **P2** | **P1** | **P1'** | **P2'** |
| | - | - | C o rD | K | D | - |
| | - | - | C | K | H or Y | - |
| | - | C | R | K | - | - |
| | - | R | R | H or R | - | - |

## Claims

1. A method for determining, monitoring and/or controlling the quality of a sample comprising at least one peptide and/or protein comprising the steps of
a) providing at least one protease-sensitive standard and/or at least one modification-sensitive standard and/or at least one standard for monitoring peptide loss,
b) adding said sample comprising at least one peptide and/or protein, and
c) determining the level of proteolytic cleavage and/or modification and/or peptide loss of said standard.

2. A method for determining, monitoring and/or controlling the quality of a sample comprising at least one peptide and/or protein comprising the steps of
a) providing said sample comprising at least one peptide and/or protein,
b) adding at least one protease-sensitive standard and/or at least one modification-sensitive standard and/or at least one standard for monitoring peptide loss, and
c) determining the level of proteolytic cleavage and/or modification and/or peptide loss of said standard.

3. The method according to claim 1 or 2 further comprising the steps of determining at at least a second point of time the level of proteolytic cleavage and/or modification and/or peptide loss of said standard and comparing the level of proteolytic cleavage and/or modification and/or peptide loss between said first and said at least second determination.

4. The method according to claim 3 for determining the progression of proteolytic cleavage and/or modification and/or peptide loss of said standard with time.

5. The method according to claim 1 or 2 further comprising the step of comparing the level of proteolytic cleavage and/or modification and/or peptide loss in said sample with the level of proteolytic cleavage and/or modification and/or peptide loss in at least one further sample.

6. The method according to any one of claims 1 to 5, wherein the modification is due to the presence of a glycosidase, phosphatase, kinase, lipase and/or nuclease.

7. The method according to any one of claims 1 to 5, wherein the proteolytic cleavages is due to the presence of at least one protease selected from the group consisting of serine proteases, cysteine proteases, threonine proteases, aspartic proteases, glutamic proteases, thiol proteases, metallo proteases, exoproteases, endoproteases and proteases involved in the clotting cascade and proteases involved in the classical and/or alternative complement cascade.

8. The method according to any one of claims 1 to 7, wherein the protease sensitive standard is at least one peptide each comprising at least one cleavage site for at least one protease.

9. The method according to claim 8, wherein the at least one cleavage site is independently a cleavage site for a protease selected from the group consisting of serine proteases, cysteine proteases, threonine proteases, aspartic proteases, glutamic proteases, thiol proteases, metallo proteases, exoproteases, endoproteases, proteases involved in the clotting cascade and proteases involved in the classical and/or alternative complement cascade.

10. The method according to any one of claims 1 to 6, wherein the modification-sensitive standard is at least one peptide comprising at least one phosphorylated amino acid residue and/or at least one recognition sequence for a kinase and/or at least one glycosylated amino acid residue and/or at least one amino acid residue with an attached lipid moiety.

11. The method according to any one of the preceding claims, wherein the at least one standard comprises a standard sensitive to proteolytic activity, modification activity and/or peptide loss activity typically present in or suspected to be present in the sample.

12. The method according to any one of the preceding claims, wherein the at least one standard comprises a standard which is derived from the same or a similar source as the sample.

13. The method according to claim 12, wherein said source is selected from the group consisting of serum, plasma, whole blood, urine, thrombocytes, leukocytes, erythrocytes, bacteria, yeasts, fungi, viruses and eukaryotic microorganisms.

14. The method according to any one of the preceding claims, wherein the at least one standard comprises a label.

15. The method according to claim 14, wherein the label is selected from the group consisting of fluorescent labels, chromophore labels, luminescent labels, radioisotopic labels, isotopic labels, isobaric labels, enzyme labels, particle labels, labels comprising nucleic acids and tag labels.

16. The method according to any one of the preceding claims, wherein the at least one standard comprises a standard derived from a species different to the species from which the sample is derived.

17. The method according to any one of the preceding claims, wherein the at least one standard comprises a standard prepared synthetically or isolated from a natural source.

18. The method according to claim 17, wherein the standard is prepared recombinantly.

19. The method according to any one of the preceding claims, wherein the at least one standard comprises a standard which is associated with at least one peptide and/or protein of the sample.

20. The method according to claim 19, wherein the association is by covalent bonding or physical interaction.

21. The method according to anyone of claims 1 to 18, wherein the at least one standard comprises a standard which is associated with the surface of the compartment comprising said sample and said at least one standard.

22. The method according to claim 21, wherein the association is by covalent bonding or physical interaction.

23. The method according to any of the preceding claims, wherein the sample is or is derived from whole blood, serum, plasma, liquor cerebrospinalis or urine.

24. The method according to any one of the previous claims, wherein the sample is to be analyzed by a method selected from the group consisting of mass spectrometry, SELDI, nuclear magnetic resonance, plasmon resonance, fluorometry, photometry, luminometry, radioactive measurements, enzymatic measurements, microscopy, immunological methods and molecular biological methods.

25. The method according to any one of the preceding claims, wherein the level of proteolytic cleavage and/or modification and/or peptide loss of said standard is determined by means different to that used for analyzing the sample.

26. The method according to any one of claims 1 to 24, wherein the level of proteolytic cleavage and/or modification and/or peptide loss of said standard is determined by means used for analyzing the sample.

27. The method according to any one the preceding claims wherein the level of proteolytic cleavage and/or modification and/or peptide loss of said standard is determined prior to, after or concomitant with the analysis of the sample.

28. The method according to any one of the preceding claims further including the step of correcting the results obtained from analyzing the sample based on the determined level of proteolytic cleavage and/or modification and/or peptide loss of said standard.

29. The method according to any one of the preceding claims further including the steps of analyzing at least two different samples and normalizing the results obtained from analyzing the samples based on the determined level of proteolytic cleavage and/or modification and/or peptide loss of said standard contained in said samples.

30. The method according to any one of the preceding claims, wherein the level of proteolytic cleavage and/or modification and/or peptide loss of said standard is determined after storing the sample, in particular freezing the sample.
